# FASCICULE DE BREVET EUROPEEN

(11) **EP 0 861 067 B1**
(45) Date de publication et mention de la délivrance du brevet: **10.04.2002**
(21) Numéro de dépôt: 96933492.9
(22) Date de dépôt: 04.10.1996
(51) Int. Cl.: A61K 9/00

(54) **FORME GALENIQUE UNITAIRE POUR HORMONOTHERAPIE LOCALE DE LA SECHERESSE VAGINALE**
GALENISCHE EINZELDOSIERUNG ZUR LOKALEN HORMONTHERAPIE DER VAGINALEN TROCKENHEIT
UNIT GALENICAL FORM FOR LOCAL HORMONOTHERAPY OF VAGINAL DRYNESS

(30) Priorité: 05.10.1995 FR 9511732
(43) Date de publication de la demande: 02.09.1998
(73) Titulaire: LABORATOIRE INNOTHERA Société Anonyme, 94111 Arcueil (FR)
(72) Inventeur: MEIGNANT, Catherine, F-75010 Paris (FR)
(74) Mandataire: Dupuis-Latour, Dominique
(86) Numéro de dépôt international: FR9601555
(87) Numéro de publication internationale: WO9712600

(56) Documents cités:
- EP-A- 0 103 995
- US-A- 5 019 395

## Description

L'invention concerne une spécialité pharmaceutique pour le traitement local, essentiellement non systémique, de la sécheresse vaginale.

On connaît les inconvénients de la sécheresse vaginale, notamment chez la femme ménopausée : dyspareunie, atrophie urogénitale pouvant entraîner des perturbations de la fonction urinaire, risques d'infection en raison d'une flore insuffisamment développée.

L'un des buts de l'invention est de proposer une spécialité convenant à un traitement essentiellement non systémique, qui se distingue donc des traitements d'hormonothérapie substitutive, où l'hormone peut être administrée *per os,* par voie transcutanée ou par administration intravaginale.

À l'opposé, les EP-A-0 103 995 et US-A-5 019 395 décrivent des formes galéniques pour hormonothérapie par voie générale où les compositions sont très fortement dosées en principe actif, respectivement de 4 à 15 % en poids, et de 0,1 à 8 % en poids, de la spécialité.

La spécialité de l'invention se distingue en particulier des spécialités d'hormonothérapie substitutive administrées par voie vaginale, par exemple sous forme de crèmes, comprimés ou suppositoires vaginaux, qui sont fortement dosées en estrogènes. Il s'agit simplement dans ce cas de tirer parti du meilleur passage systémique de la voie vaginale par rapport à la voie orale, compte tenu notamment d'une absence de métabolisation des estrogènes lorsque l'on utilise cette voie.

L'invention cherche au contraire à réaliser un traitement local, avec passage systémique minime ou nul, par apport direct d'un estrogène naturel, et plus particulièrement du 17β-estradiol, permettant de soulager les troubles locaux en évitant les effets secondaires systémiques susceptibles d'apparaître chez certaines patientes, notamment l'apparition d'une hyperplasie endométriale.

On a déjà proposé des traitements locaux de ce type, par exemple sous forme d'un anneau vaginal en forme de tore enfermant un estrogène qui diffuse au travers de la membrane poreuse de l'anneau, permettant ainsi une libération continue sur une longue durée.

Ces anneaux présentent cependant l'inconvénient, comme tout dispositif intravaginal, d'une part d'impliquer la présence dans l'organisme d'un corps étranger non dégradable et d'autre part de nécessiter des manipulations pour sa pose et son retrait.

n a été également proposé pour ce traitement local une forme galénique comportant du 17β-estradiol sous forme de comprimés vaginaux administrés quotidiennement. Ces comprimés sont des comprimés matriciels comprenant un excipient tel qu'un polymère cellulosique absorbant les traces d'humidité vaginale résiduelle de manière à imprégner la matrice contenant le principe actif et à libérer celui-ci de façon progressive.

Toutefois, du fait de leur forme galénique particulière, le dosage de ces comprimés doit être relativement élevé pour procurer les résultats voulus, typiquement un dosage de 25 µg de 17β-estradiol par comprimé (un comprimé correspondant à une dose unitaire) pour permettre l'amélioration cytologique, histologique et clinique recherchée de la muqueuse vaginale. Du fait de cette dose relativement élevée, on note d'après les études cliniques une prolifération de l'endomètre chez certaines patientes, signe d'un passage systémique du 17β-estradiol: voir notamment C. Felding et coll., Preoperative Treatment with OEstradiol in Women Scheduled for Vaginal Operation for Genital Prolapse. A Randomised, Double-Blind Trial, *Maturitas,* 1992, *15*, 241-249.

L'un des buts de la présente invention est de proposer une spécialité du type précité, avec une forme galénique particulière permettant de réduire le dosage du 17β-estradiol de manière à éviter le passage systémique malgré l'extrême sensibilité des muqueuses vaginales aux estrogènes, mais tout en procurant une efficacité trophique satisfaisante.

Selon l'invention, cette spécialité est caractérisée par une forme galénique unitaire comprenant un estrogène naturel choisi parmi le 17β-estradiol et ses sels en solution ou en suspension dans un agent lipophile, avec une teneur en estrogène correspondant à une dose unitaire équivalente d'au plus 15 µg, de préférence moins de 10 µg, de 17β-estradiol, un agent hydrophile gélifiant bioadhésif, un agent gélifiant de l'agent lipophile et un agent hydrodispersible.

Au contact des sécrétions vaginales, l'agent hydrophile bioadhésif se gélifie et, grâce à la présence de l'agent hydrodispersible, la forme galénique s'émulsionne, ce qui permet notamment une diffusion passive directe du principe actif entre l'excipient émulsionné et la muqueuse vaginale avec laquelle celui-ci est en contact. Le caractère bioadhésif (plus précisément, mucoadhésif) du gélifiant hydrophile permet de rendre l'émulsion collante sur la muqueuse, avec un faible écoulement, procurant ainsi un maintien de longue durée.

Ce maintien de longue durée permet en particulier d'espacer les applications, qui peuvent n'être que quotidiennes, ou encore moins fréquentes (notamment en phase d'entretien).

L'estrogène, avantageusement micronisé, peut être présent dans la forme galénique soit sous forme libre soit sous forme vectorisée, notamment par encapsulation dans des vecteurs de type nanoparticules tels que des biovecteurs supramoléculaires.

Dans une première mise en oeuvre, la spécialité est réalisée sous forme de capsule comprenant, d'une part, une enveloppe externe solide dure ou molle contenant de la gélatine et, d'autre part, une phase interne liquide ou semi-liquide non aqueuse contenant l'agent lipophile avec l'estrogène en solution ou en suspension, l'agent hydrophile gélifiant bioadhésif, l'agent gélifiant de l'agent lipophile et l'agent hydrodispersible.

Il peut s'agir d'une capsule dure (gélule) ou, avantageusement, d'une capsule molle, c'est-à-dire dans laquelle l'enveloppe externe contient de la glycérine.

Dans ce dernier cas, avantageusement :
- l'agent lipophile est choisi parmi les triglycérides liquides ;
- l'agent hydrophile gélifiant bioadhésif est choisi parmi les acides carboxyvinyliques, l'hydroxypropylcellulose, la carboxyméthylcellulose, la gélatine, la gomme xanthane, la gomme Guar, le silicate d'aluminium et leurs mélanges ;
- l'agent gélifiant de l'agent lipophile est la silice colloïdale hydrophobe ;
- l'agent hydrodispersible est choisi parmi les polyoxyéthylène glycols, le 7-glycéryl-cocoate de polyoxyéthylène glycol et leurs mélanges.
- la composition de la phase interne est : 17β-estradiol micronisé libre ou vectorisé 2,5 à 15 µg; hydroxypropylcellulose, 120 mg ; silice colloïdale hydrophobe, 50 à 80 mg ; 7-glycéryl-cocoate de polyoxyéthylène glycol, 400 mg ; triglycérides liquides, q.s. 1600 mg.

Dans une autre mise en oeuvre, la spécialité est réalisée sous forme d'ovules à libération prolongée, comprenant une phase homogène solide dure ou semi-molle non aqueuse contenant l'agent lipophile avec l'estrogène en solution ou en suspension, l'agent hydrophile gélifiant bioadhésif, l'agent gélifiant de l'agent lipophile et l'agent hydrodispersible.

Dans ce cas, avantageusement :
- l'agent lipophile est choisi parmi les triglycérides solides à point de fusion d'environ 35° C, la cire de Carnauba, le beurre de cacao et leurs mélanges ;
- l'agent hydrophile gélifiant bioadhésif est choisi parmi les acides carboxyvinyliques, l'hydroxypropylcellulose, la carboxyméthylcellulose, la gélatine, la gomme xanthane, la gomme Guar, le silicate d'aluminium et leurs mélanges ;
- l'agent gélifiant de l'agent lipophile est la silice colloïdale hydrophobe ;
- l'agent hydrodispersible est choisi parmi les polyoxyéthylène glycols et leurs mélanges ;
- la composition est : 17β-estradiol micronisé libre ou vectorisé 2,5 à 15 µg; hydroxypropylcellulose, 80 mg; silice colloïdale hydrophobe, 5 à 60 mg ; polyoxyéthylène glycol, 50 à 200 mg; acide carboxyvinylique, 8 mg ; triglycérides solides q.s. 1600 mg.

Ces formulations, dans un cas ou dans l'autre présentent de nombreux avantages :
- elle sont bien tolérées, stables et galéniquement acceptables,
- elle procurent une bioadhésion permettant d'éviter au maximum le phénomène d'écoulement,
- elles assurent la compatibilité des véhicules avec le principe actif,
- elles favorisent l'émulsification du véhicule contenant le principe actif avec les sécrétions vaginales en apportant un certain caractère hydrophile.

On va maintenant décrire plus en détail les différents aspects de la présente invention, en donnant ensuite divers exemples de formulations.

### Choix du principe actif

On choisit pour la spécialité de l'invention un estrogène choisi parmi le 17β-estradiol, ses sels et ses dérivés.

Ce groupe comprend la famille de composés dont la structure chimique répond à la formule générale :

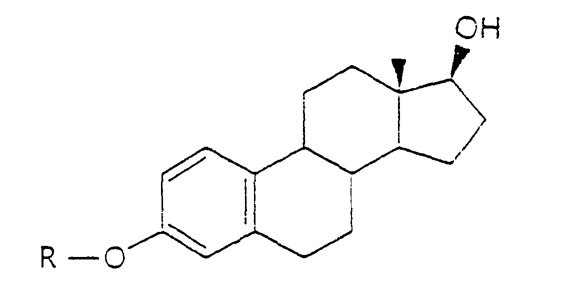

Lorsque R = H, le composé est le 17β-estradiol, qui est l'hormone naturelle produite physiologiquement par les ovaires des femmes fertiles et dont la carence est responsable des troubles fonctionnels ressentis chez la patiente ménopausée.

Le 17β-estradiol est un agoniste estrogénique physiologique. Son rôle trophique sur la muqueuse vulvo-vaginale est reconnu et a été largement décrit, de même que la réversibilité des troubles histologiques fonctionnels et cliniques par administration de 17β-estradiol. L'estradiol et ses dérivés (sels) diminuent le pH vaginal et augmentent la différence de potentiel transvaginal, la quantité de sécrétions vaginales et le débit sanguin local.

Chez la femme, des récepteurs à haute affinité pour l'estradiol ont été décelés au niveau de l'épithélium vaginal. Ceux-ci présentent pour l'estradiol radiomarqué une affinité voisine de celle calculée pour les récepteurs du myomètre mais leur nombre paraît plus faible. Ces récepteurs se caractérisent par une affinité décroissante pour les composés suivants : 17β-estradiol > estriol > estrone.

Mais alors que le 17β-estradiol se révèle être un agoniste pur, l'estriol, son métabolite naturel, se caractérise par des propriétés d'agoniste partiel, voire d'antagoniste. Un effet antagoniste pourrait se manifester vis à vis de l'estrogène naturel.

Un traitement par le 17β-estradiol présente donc l'avantage de l'additivité des effets en présence de l'estradiol endogène, alors qu'un traitement par l'estriol présente l'inconvénient d'un effet antagoniste vis-à-vis de l'estradiol endogène. De plus, l'estriol, du fait de son activité intrinsèque plus faible que celle du 17β-estradiol, se révèle moins actif que celui-ci (certains auteurs expliquent ce phénomène par une plus grande vitesse de dissociation de ses récepteurs nucléaires).

Comme l'activité présentée par un agoniste partiel est plus dépendante du nombre de récepteurs que celle de l'agoniste complet, la différence d'activité entre ces deux composés estrogéniques est d'autant plus marquée que le taux de récepteurs tissulaires vaginaux paraît plus faible qu'au niveau du myomètre.

En conclusion, vu la haute affinité du 17β-estradiol pour les récepteurs estrogéniques vaginaux et surtout son profil d'activité en tant qu'agoniste complet, cet estrogène naturel constitue un meilleur choix que l'estriol pour obtenir un effet trophique local.

Les mêmes remarques s'appliquent à l'estrone, qui est un précurseur de l'estriol, ainsi qu'aux dérivés de synthèse de l'estradiol tels que notamment le diétheroxyde d'estradiol (promestriène DCI).

### Choix du dosage

Le dosage doit être choisi de manière à soulager les troubles locaux en évitant au maximum l'absorption transvaginale.

Pour atteindre ces objectifs, on choisit une dose de 10 µg de 17β-estradiol, correspondant à une dose unitaire (administration journalière unique, voire encore moins fréquente).

Lorsque le 17β-estradiol est présent à cette dose sous forme micronisée libre, on n'observe qu'un très léger passage systémique, sous forme d'un simple pic plasmatique au bout d'une heure environ après l'administration ; la concentration plasmatique maximale du pic ne dépasse jamais 30 pg/ml, mais elle est bien évidemment très fugace.

Si l'on souhaite néanmoins éviter ce très faible passage, une première solution consiste à réduire la teneur en principe actif, typiquement à des doses de 5 µg ou même 2,5 µg par administration unitaire.

Une autre possibilité consiste, en conservant le même excipient, à vectoriser le principe actif au lieu de l'y mettre sous forme libre.

On va expliquer ci-dessous l'intérêt de cette vectorisation et la manière dont on peut la réaliser.

### Vectorisation du 17β-estradiol

L'un des buts de la vectorisation du 17β-estradiol est d'éliminer l'éventuel passage systémique du principe actif par une libération plus progressive de ce dernier qui aura pour effet d"'étaler" le pic plasmatique en réduisant son amplitude maximale, qui pourra rester toujours inférieure à 50 pg/ml de concentration plasmatique.

Avantageusement, la vectorisation permettra également d'augmenter la durée d'action locale du principe actif.

Ces deux buts peuvent être atteints de la manière suivante.

Pour éviter tout passage systémique, la taille du vecteur doit être suffisamment élevée pour ne pas traverser l'épithélium vaginal. Une taille de l'ordre de 200 nm de diamètre répond à ce critère. Bien entendu, le vecteur doit être compatible avec le 17β-estradiol, permettre son relargage progressif, doit être compatible avec le mucus vaginal et parfaitement toléré.

Pour augmenter la durée d'action, on peut choisir un système de bioadhésion par interactions électrostatiques. En effet, dans des conditions normales, le mucus vaginal est de nature acide (pH de l'ordre de 4) tandis qu'à la ménopause ce pH a tendance à augmenter aux environs de 6. Il est donc intéressant d'apporter en périphérie du vecteur des charges positives qui peuvent ainsi interagir avec les charges négatives du mucus.

Il faut noter que les propriétés acides requises pour une interaction maximale entre le mucus et le vecteur sont réduites en période de ménopause (pH de l'ordre de 6), mais ces conditions de faible acidité peuvent être suffisantes pour une interaction efficace avec les vecteurs.

Un vecteur répondant à ces différentes conditions est par exemple constitué par des nanoparticules (c'est-à-dire des particules dont le diamètre est de l'ordre de quelques dizaines ou au plus quelques centaines de nanomètres) tels que les "biovecteurs supramoléculaires" (BVSM) décrits dans le WO-A-89/11271 (Centre national de la recherche scientifique) et produits par la société Biovector Therapeutics SA.

Ces BVSM, qui sont des vecteurs en eux-mêmes connus, comprennent un noyau hydrophile non liquide, une enveloppe interne de nature lipidique liée au noyau par des liaisons covalentes et une enveloppe externe amphiphile liée à l'enveloppe interne lipidique par des interactions hydrophobes.

Ces vecteurs peuvent être chargés en principe actif, ici par le 17β-estradiol (qui est lipophile) encapsulé dans le vecteur, l'ensemble constituant alors un transporteur de principe actif biomimétique des systèmes de transport endogènes telles que les lipoprotéines.

### Exemple de formulation d'une capsule molle

Pour satisfaire au concept de bioadhésion de la forme galénique de l'invention et éviter au maximum le phénomène d'écoulement, la phase interne de cette capsule molle renferme dans cet exemple des polymères bioadhésifs hydrophiles gélifiants biocompatibles ayant la propriété d'incorporer au maximum l'humidité des sécrétions vaginales pour augmenter la viscosité et prolonger ainsi le maintien *in situ* de l'émulsion.

Par ailleurs, le phénomène d'écoulement du contenu lipophile de la phase interne de la capsule est évité par l'emploi d'un agent gélifiant de cet agent lipophile. Dans cet exemple, au moins l'un des ingrédients de la phase interne favorise l'émulsification avec les sécrétions vaginales du dérivé lipophile qui est le constituant essentiel de la phase grasse.

Une composition typique de la phase interne est la suivante :

| | |
|---|---|
| 17β-estradiol micronisé libre ou vectorisé | 2,5 à 15 µg |
| | (soit 1,5625 à 9,375 ppm) |
| Hydroxypropylcellulose (Klucel® HXF) | 120 mg |
| Silice colloïdale hydrophobe (Aérosil® R972) | 70 mg |
| 7-glycéryl-cocoate de polyoxyéthylène glycol (Cétiol® HE) | 400 mg |
| Triglycérides liquides (Miglyol® 812) | q.s. 1600 mg |

On notera la très faible concentration finale en principe actif, qui est de 1,5625.10⁻⁶ à 9,375.10⁻⁶ pour la plage de doses unitaires indiquée ci-dessus, en particulier de 6,25.10⁻⁶ (0,000625 %) dans le cas correspondant aux essais cliniques dont on rendra compte plus bas.

Cette phase interne est introduite une fois préparée dans une enveloppe externe comportant de la gélatine/glycérine et correspondant à une structure de capsule molle.

Diverses variations de dosage des excipients peuvent être envisagées. Ainsi, la dose de silice hydrophobe peut être comprise entre 50 et 80 mg.

Il est également possible de modifier la composition des excipients.

On peut ainsi remplacer le polymère hydrophile gélifiant bioadhésif (hydroxypropylcellulose) par d'autres composants hydrophiles gélifiants bioadhésifs tels que : acides carboxyvinyliques, hydroxypropylcellulose, carboxyméthylcellulose, gélatine, gomme xanthane, gomme Guar, silicate d'aluminium ou un mélange de deux ou plusieurs des composants précédents.

Quant à l'agent hydrodispersible, le 7-glycéryl-cocoate de polyoxyéthylène glycol peut être remplacé par un polyoxyéthylèneglycol (PEG).

### Exemple de formulation d'un ovule à libération prolongée

Dans ce cas, la spécialité comporte une phase homogène solide dure ou semi-molle dont la composition typique est la suivante :

| | |
|---|---|
| 17β-estradiol micronisé libre ou vectorisé | 2,5 à 15 µg |
| Hydroxypropylcellulose (Klucel® HXF) | 80 mg |
| Silice colloïdale hydrophobe (Aérosil® R 972) | 40 mg |
| Polyoxyéthylène glycol (PEG 400) | 80 mg |
| Acide carboxyvinylique (Carbopol® 974 P) | 8 mg |
| Triglycérides solides (Witespol® S 51) | q.s. 1600 mg |

Diverses variations de dosage des excipients peuvent être envisagées. Ainsi, la dose de silice colloïdale hydrophobe peut être comprise entre 5 et 60 mg, et celle du PEG entre 50 et 200 mg.

Il est également possible de modifier la composition des excipients.

Ainsi, le Witespol® S 51 peut être remplacé par de la cire de Carnauba, du beurre de cacao ou autres triglycérides à point de fusion d'environ 35°C, par exemple de type Ovucire® .

Les polymères hydrophiles gélifiants bioadhésifs (Klucel® et Carbopol® ) peuvent être remplacés par les mêmes substituants que ceux indiqués plus haut dans l'exemple d'une formulation de capsule molle.

Par ailleurs, le PEG 400 peut être remplacé par un PEG 200 à 4000, en proportions adaptées.

### Essais cliniques

Les résultats obtenus sur six patientes mettent en évidence les éléments suivants.

Tolérance clinique et biologique : Dans les conditions de l'essai, la tolérance clinique locale et générale de la formulation ci-dessus selon l'invention, présentée sous forme de capsules molles dosées à 2,5 µg, 5 µg et 10 µg a été excellente. Aucun événement indésirable n'a été rapporté. La tolérance biologique a été excellente. Aucune anomalie ayant une signification clinique n'a été rapportée.

Analyse pharmacocinétique : Du point de vue pharmacocinétique, les concentrations plasmatiques d'estradiol demeurent non quantifiables chez l'ensemble des sujets après administration des doses faibles (2,5 et 5 µg) et chez la moitié des sujets à la dose forte (10 µg). Chez les trois autres sujets, des taux d'estradiol supérieurs à la limite de quantification n'ont été mesurés après traitement que dans quelques échantillons (2 ou 3) et ne dépassent pas 30 pg/ml.

En ce qui concerne l'estrone, les concentrations mesurées après traitement sont en général du même ordre de grandeur que celles mesurées avant traitement. En effet, lorsque les taux d'estrone sont plus élevés après traitement (2 ou 3 sujets par groupe selon le groupe), la concentration la plus forte ne dépasse pas de plus de 22 % (sujet n° 02), 34 % (sujet n° 06) et 26 % (sujet n° 06) les valeurs mesurées avant traitement, à 2,5 µg, 5 µg et 10 µg de 17β-estradiol respectivement. Dans tous les cas, les concentrations d'estrone ne dépassent jamais 30 pg/ml. L'examen des profils de concentrations plasmatiques d'estrone montre qu'il n'existe pas de proportionnalité entre les Cₘₐₓ ou les SSC et la dose administrée.

Conclusion générale : Après administration vaginale unique d'une capsule molle dosée à 2,5 µg, à 5 µg et à 10 µg de 17β-estradiol, la tolérance clinique a été excellente pour l'ensemble des six sujets inclus dans l'essai. La tolérance biologique a été également excellente. Aucune anomalie ayant une signification clinique n'a été rapportée.

Du point de vue pharmacocinétique, la résorption vaginale de l'estradiol est nulle après administration des capsules dosées à 2,5 et 5 µg de 17β-estradiol. Après administration de la capsule dosée à 10 µg de 17β-estradiol, l'estradiol reste indétectable au niveau plasmatique chez trois sujets sur six. Pour les autres sujets, quelques concentrations plasmatiques d'estrone montrent que les taux mesurés après traitement sont comparables aux taux mesurés avant traitement. On peut donc conclure de cette étude que l'absorption vaginale de l'estradiol à partir des capsules molles dosées de 2,5 µg à 10 µg de 17β-estradiol est quasiment nulle sur la gamme des doses testées.

On notera en particulier l'absence de pic supérieur à 50 pg/ml, limite au-delà des effets secondaires pourraient apparaître chez certains sujets (*supra*). La forme libre, micronisée, du 17β-estradiol se révèle ici pleinement satisfaisante, et ne nécessite pas le recours à une forme vectorisée pour éviter le dépassement du seuil de 50 pg/ml. Cette forme vectorisée serait cependant envisageable, si l'on souhaitait prolonger le temps d'action du principe actif.

## Revendications

1. Une spécialité pharmaceutique pour traitement local, essentiellement non systémique, de la sécheresse vaginale, notamment chez la femme ménopausée, **caractérisée par** une forme galénique unitaire comprenant un estrogène naturel choisi parmi le 17β-estradiol et ses sels en solution ou en suspension dans un agent lipophile, avec une teneur en estrogène correspondant à une dose unitaire équivalente d'au plus 15 µg, de préférence moins de 10 µg, de 17β-estradiol, un agent hydrophile gélifiant bioadhésif, un agent gélifiant de l'agent lipophile et un agent hydrodispersible.

2. La spécialité de la revendication 1, dans laquelle l'estrogène est présent sous forme libre, avantageusement micronisée.

3. La spécialité de la revendication 1, dans laquelle l'estrogène est présent sous forme vectorisée, avantageusement micronisée.

4. La spécialité de la revendication 3, dans laquelle l'estrogène est vectorisé par encapsulation dans des vecteurs de type nanoparticules.

5. La spécialité de la revendication 4, dans laquelle l'estrogène est vectorisé par encapsulation dans des vecteurs particulaires de type biovecteurs supramoléculaires.

6. La spécialité de la revendication 1, sous forme de capsule comprenant, d'une part, une enveloppe externe solide dure ou molle contenant de la gélatine et, d'autre part, une phase interne liquide ou semi-liquide non aqueuse contenant l'agent lipophile avec l'estrogène en solution ou en suspension, l'agent hydrophile gélifiant bioadhésif, l'agent gélifiant de l'agent lipophile et l'agent hydrodispersible.

7. La spécialité de la revendication 6, sous forme de capsule molle, dans laquelle l'enveloppe externe contient de la glycérine.

8. La spécialité de la revendication 6, dans laquelle l'agent lipophile est choisi parmi les triglycérides liquides.

9. La spécialité de la revendication 6, dans laquelle l'agent hydrophile gélifiant bioadhésif est choisi parmi les acides carboxyvinyliques, l'hydroxypropylcellulose, la carboxyméthylcellulose, la gélatine, la gomme xanthane, la gomme Guar, le silicate d'aluminium et leurs mélanges.

10. La spécialité de la revendication 6, dans laquelle l'agent gélifiant de l'agent lipophile est la silice colloïdale hydrophobe.

11. La spécialité de la revendication 6, dans laquelle l'agent hydrodispersible est choisi parmi les polyoxyéthylène glycols, le 7-glycéryl-cocoate de polyoxyéthylène glycol et leurs mélanges.

12. la spécialité de la revendication 6, dans laquelle la composition de la phase interne est :
| | |
|---|---|
| 17β-estradiol micronisé libre ou vectorisé | 2,5 à 15 µg |
| Hydroxypropylcellulose | 120 mg |
| Silice colloïdale hydrophobe | 50 à 80 mg |
| 7-glycéryl-cocoate de polyoxyéthylène glycol | 400 mg |
| Triglycérides liquides | q.s. 1600 mg |

13. La spécialité de la revendication 1, sous forme d'ovule à libération prolongée comprenant une phase homogène solide dure ou semi-molle non aqueuse contenant l'agent lipophile avec l'estrogène en solution ou en suspension, l'agent hydrophile gélifiant bioadhésif, l'agent gélifiant de l'agent lipophile et l'agent hydrodispersible.

14. La spécialité de la revendication 13, dans laquelle l'agent lipophile est choisi parmi les triglycérides solides à point de fusion d'environ 35° C, la cire de Carnauba, le beurre de cacao et leurs mélanges.

15. La spécialité de la revendication 13, dans laquelle l'agent hydrophile gélifiant bioadhésif est choisi parmi les acides carboxyvinyliques, l'hydroxypropylcellulose, la carboxyméthylcellulose, la gélatine, la gomme xanthane, la gomme Guar, le silicate d'aluminium et leurs mélanges.

16. La spécialité de la revendication 3, dans laquelle l'agent gélifiant de l'agent lipophile est la silice colloïdale hydrophobe.

17. La spécialité de la revendication 13, dans laquelle l'agent hydrodispersible est choisi parmi les polyoxyéthylène glycols et leurs mélanges.

18. la spécialité de la revendication 13, dont la composition est :
| | |
|---|---|
| 17β-estradiol micronisé libre ou vectorisé | 2,5 à 15 µg |
| Hydroxypropylcellulose | 80 mg |
| Silice colloïdale hydrophobe | 5 à 60 mg |
| Polyoxyéthylène glycol | 50 à 200 mg |
| Acide carboxyvinylique | 8 mg |
| Triglycérides solides | q.s. 1600 mg |

## Patentansprüche

1. Pharmazeutische Spezialität zur Behandlung von lokaler, im wesentlichen nicht systemischer vaginaler Trockenheit, insbesondere bei Frauen in der Menopause, **gekennzeichnet durch** eine galenische Form, die ein natürliches Oestrogen, ausgewählt unter 17β-Oestradiol und seinen Salzen, in Lösung oder in Suspension in einem lipophilen Agens, mit einem Gehalt an Oestrogen entsprechend einer Einheitsdosis, die höchstens 15 µg, vorzugsweise weniger als 10 µg, 17β-Oestradiol äquivalent ist, ein hydrophiles bioadhäsives Geliermittel, ein Geliermittel für das lipophile Agens und ein hydrodispergierbares Agens enthält.

2. Spezialität nach Anspruch 1, wobei das Oestrogen in freier Form, vorzugsweise mikronisiert vorliegt.

3. Spezialität nach Anspruch 1, wobei das Oestrogen in vektorisierter Form. vorzugsweise mikronisiert vorliegt.

4. Spezialität nach Anspruch 3, wobei das Oestrogen durch Einkapseln in Vektoren von Nanopartikel-Typ vektorisiert ist.

5. Spezialität nach Anspruch 4, wobei das Oestrogen durch Einkapseln in partikuläre Vektoren vom Typ supramolekulare Biovektoren vektorisiert ist.

6. Spezialität nach Anspruch 1 in Form von Kapseln, die einerseits eine äußere feste, harte oder weiche, Gelantine enthaltende Hülle und andererseits eine innere, flüssige oder halbflüssige nicht-wässrige Phase aufweisen, die das lipophile Agens mit Oestrogen in Lösung oder in Suspension, das hydrophile bioadhäsive Geliermittel, das Geliermittel für das lipophile Agens und das hydrodispergierbare Agens enthält.

7. Spezialität nach Anspruch 6, in Form einer weichen Kapsel bei der die äußere Hülle Glicerin enthält.

8. Spezialität nach Anspruch 6, wobei das lipophile Agens unter flüssigen Trigliceriden ausgewählt ist.

9. Spezialität nach Anspruch 6, wobei das hydrophile bioadhesive Geliermittel ausgewählt ist unter Vinylcarbonsäuren, Hydroxypropylcellulose, Carboxymethylcellulose, Gelantine, Xanthangummen, Guargummen, Aluminiumsilikat und Gemischen hiervon.

10. Spezialität nach Anspruch 6, wobei das Geliermittel für das lipophile Agens kolloidales hydrophobes Siliciumdioxid ist.

11. Spezialität nach Anspruch 6, wobei das hydrodispergierbare Agens ausgewählt ist unter Polyoxyethylenglykolen, 7-Glycerylcocoat von Polyoxyethylenglykol und Gemischen hiervon.

12. Spezialität nach Anspruch 6, wobei die Zusammensetzung der inneren Phase wie folgt ist:
| | |
|---|---|
| mikronisiertes 17β-Oestradiol, in freier oder vektorisierter Form | 2,5 bis 15 µg |
| Hydoxypropylcellulose | 120 mg |
| kolloidales hydophobes Siliciumdioxid | 50 bis 80 mg |
| 7-Glycerylcocoat von Polyethylenglykol | 400 mg |
| flüssige Triglyceride | Rest zu 1600 mg. |

13. Spezialität nach Anspruch 1, in Form von Kugeln mit verlängerter Freisetzung, aufweisend eine homogene feste, harte oder halbweiche nicht-wässrige Phase, die das lipophile Agens mit Oestrogen in Lösung oder in Suspension, das hydrophyle bioadhäsive Geliermittel, das Geliermittel für das lipophile Agens und das hydrodispergierbare Agens enthält.

14. Spezialität nach Anspruch 13, wobei das lipophile Agens ausgewählt ist unter festen Triglyceriden mit einem Schmelzpunkt um 35°C, Carnaubawachs, Kakaobutter oder Gemischen hiervon.

15. Spezialität nach Anspruch 13, wobei das hydrophyle bioadhäsive Geliermittel ausgewählt ist unter Vinylcarbonsäuren, Hydroxypropylcellulose, Carboxymethylcellulose, Gelantine, Xanthangummen, Guargummen, Aluminiumsilikat und Gemischen hiervon.

16. Spezialität nach Anspruch 3, wobei das Geliermittel für das lipophile Agents kolloidales hydrophobes Siliciumdioxid ist.

17. Spezialität nach Anspruch 13, wobei das hydrodispergierbare Agens ausgewählt ist unter Polyethylenglykolen und Gemischen hiervon.

18. Spezialität nach Anspruch 13 mit folgender Zusammensetzung:
| | |
|---|---|
| mikronisiertes 17β-Oestradiol, in freier oder vektorisierter Form | 2,5 bis 15 µg |
| Hydroxypropycellulose | 80 mg |
| kolloidales hydophobes Siliciumdioxid | 5 bis 60 mg |
| Polyoxyethylenglykol | 50 bis 200 mg |
| Vinylcarbonsäure | 8 mg |
| feste Triclyceride | Rest zu 1600 mg. |

## Claims

1. A pharmaceutical medicament for local, essentially non systemic, treatment of vaginal dryness, in particular in the menopausal woman, **characterized by** a unit galenical formulation comprising a natural estrogen selected from 17β-estradiol and its salts in solution or in suspension in a lipophilic agent, with an estrogen content which corresponds to an equivalent unit dose of at most 15 µg, preferably less than 10 µg, of 17β-estradiol, a hydrophilic gel-forming bioadhesive agent, a gelling agent for the lipophilic agent and a hydrodispersible agent.

2. The medicament of claim 1, in which the estrogen is present in its free form, advantageously micronized.

3. The medicament of claim 1, in which the estrogen is present in its vectorized form, advantageously micronized.

4. The medicament of claim 3, in which the estrogen is vectorized by encapsulation in nanoparticle type vectors.

5. The medicament of claim 4, in which the estrogen is vectorized by encapsulation in particulate supramolecular biovector type vectors.

6. The medicament of claim 1, in capsule form comprising a hard or soft solid outer envelope containing gelatin and a non aqueous liquid or semi-liquid inner phase containing the lipophilic agent with the estrogen in solution or in suspension, the hydrophilic gel-forming bioadhesive agent, the gelling agent for the lipophilic agent and the hydrodispersible agent.

7. The medicament of claim 6, in the form of a soft capsule, in which the outer envelope contains glycerine.

8. The medicament of claim 6, in which the lipophilic agent is selected from liquid triglycerides.

9. The medicament of claim 6, in which the hydrophilic gel-forming bioadhesive agent is selected from carboxyvinylic acids, hydroxypropylcellulose, carboxymethylcellulose, gelatin, xanthane gum, guar gum, aluminum silicate and mixtures thereof.

10. The medicament of claim 6, in which the gelling agent for the lipophilic agent is hydrophobic colloidal silica.

11. The medicament of claim 6, in which the hydrodispersible agent is selected from polyoxyethylene glycols, polyoxyethylene glycol 7-glyceryl-cocoate and mixtures thereof.

12. The medicament of claim 6, in which the composition of the inner phase is:
| | |
|---|---|
| Free or vectorized 17β-estradiol | 2.5 µg to 15 µg |
| Hydroxypropylcellulose | 120 mg |
| Hydrophobic colloidal silica | 50 mg to 80 mg |
| Polyoxyethylene glycol 7-glyceryl-cocoate | 400 mg |
| Liquid triglycerides | q.s. 1600 mg |

13. The medicament of claim 1, in the form of a slow release vaginal suppository comprising a non aqueous hard or semi-soft solid homogeneous phase containing the lipophilic agent with the estrogen in solution or in suspension, the hydrophilic gel-forming bioadhesive agent, the gelling agent for the lipophilic agent and the hydrodispersible agent.

14. The medicament of claim 13, in which the lipophilic agent is selected from solid triglycerides with a melting point of about 35°C, carnauba wax, cocoa butter and mixtures thereof.

15. The medicament of claim 13, in which the hydrophilic gel-forming bioadhesive agent is selected from carboxyvinylic acids, hydroxypropylcellulose, carboxymethylcellulose, gelatin, xanthane gum, guar gum, aluminum silicate and mixtures thereof.

16. The medicament of claim 3, in which the gelling agent for the lipophilic agent is hydrophobic colloidal silica.

17. The medicament of claim 13, in which the hydrodispersible agent is selected from polyoxyethylene glycols and mixtures thereof.

18. The medicament of claim 13, in which the composition is:
| | |
|---|---|
| Free or vectorized 17β-estradiol | 2.5 µg to 15 µg |
| Hydroxypropylcellulose | 80 mg |
| Hydrophobic colloidal silica | 5 mg to 60 mg |
| Polyoxyethylene glycol | 50 mg to 200 mg |
| Carboxyvinylic acid | 8 mg |
| Solid triglycerides | q.s. 1600 mg |
